# FASCICULE DE BREVET EUROPEEN

(11) **EP 0 976 726 B1**
(45) Date de publication et mention de la délivrance du brevet: **28.05.2003**
(21) Numéro de dépôt: 99401561.8
(22) Date de dépôt: 23.06.1999
(51) Int. Cl.: C07C 319/26, C07C 321/14

(54) **Composition à base de diméthyldisulfure à odeur masquée**
Geruchsmaskierte Dimethylsulfid-Zusammensetzung
Dimethylsulphide composition with masked odour

(30) Priorité: 31.07.1998 FR 9809864
(43) Date de publication de la demande: 02.02.2000
(73) Titulaire: Atofina, 92800 Puteaux (FR)
(72) Inventeur: Fremy, Georges, 64150 Os-Marsillon (FR)
(74) Mandataire: Granet, Pierre

(56) Documents cités:
- US-A- 5 218 147
- US-A- 5 559 271

## Description

La présente invention concerne le domaine des sulfures organiques et plus particulièrement celui du diméthyldisulfure.

Le diméthyldisulfure (DMDS) présente une odeur forte et agressive due en partie à la présence d'impuretés fortement odorantes et en partie à l'odeur aillée et éthérée intrinsèque au DMDS. Cette forte odeur entrave un développement accru de ce produit dans des applications telles que la sulfuration de catalyseurs ou en tant qu'additif de charge pour le vapocraquage. Par rapport à d'autres produits utilisés dans ces applications tels que les tertioalkylpolysulfures, le DMDS présente de nombreux avantages, notamment une teneur en soufre élevée (68%) et des produits de dégradation non cokants (CH₄, H₂S). De plus, dans ces applications, le DMDS conduit à des performances généralement supérieures aux autres produits tels que les tertioalkylpolysulfures. Cependant, ces autres produits ont des niveaux odorants nettement inférieurs et rendent de ce fait leur manipulation plus aisée comparativement au DMDS.

Parmi les méthodes de synthèse du DMDS, une méthode particulièrement efficace et économique est l'oxydation du méthylmercaptan par le soufre selon la réaction:

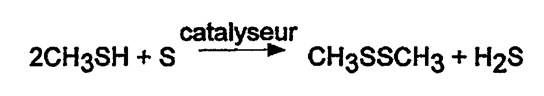

Cette oxydation du méthylmercaptan par le soufre, catalysée par des agents basiques organiques ou inorganiques, homogènes ou hétérogènes, en discontinu ou en continu, s'accompagne d'un dégagement d'hydrogène sulfuré ainsi que de diméthylpolysulfures (CH₃SₓCH₃) de rang en soufre x supérieur à 2. Pour fabriquer le DMDS selon ce procédé avec des rendements élevés et une production limitée en DMPS (diméthylpolysulfures de rang supérieur à 2), le brevet européen 0 446 109 décrit un procédé de préparation comprenant deux zones de réaction interrompues par une zone de dégazage intermédiaire et suivies d'une zone de distillation. Bien que performant en termes de rendement et de sélectivité en DMDS, il s'avère que ce procédé conduit à laisser dans le produit fini une quantité non négligeable de méthylmercaptan (environ 4000 ppm) ainsi qu'une très faible quantité de diméthylsulfure (environ 300 ppm) provenant du méthylmercaptan utilisé ou produit lors de la synthèse du DMDS. La résultante de ces impuretés volatiles est qu'elles conduisent à rendre très désagréable et agressive l'odeur du DMDS et cette forte odeur est considérée comme une gêne importante lors de la manipulation de ce produit par les utilisateurs.

Pour masquer l'odeur de polysulfures organiques, le brevet US 5 559 271 préconise de leur ajouter une certaine quantité de produit masquant tel que, notamment, la vanilline ou l'éthylvanilline. Bien que sa formule générale inclue le DMDS, ce brevet vise plus particulièrement le traitement des polysulfures lourds comme, par exemple, le pentasulfure de di-t-nonyle. L'application de cette méthode au DMDS ne permet pas de masquer son odeur nauséabonde et très désagréable.

Il a maintenant été trouvé que, dans le cas spécifique du DMDS, l'addition d'un agent masquant d'odeur n'est efficace que si le DMDS utilisé présente des teneurs réduites en impuretés volatiles fortement odorantes telles que le méthylmercaptan et le diméthylsulfure et contient de préférence moins de 200 ppm en poids de méthylmercaptan et moins de 50 ppm en poids de diméthylsulfure. Il a également été trouvé que les agents masquant d'odeur les plus efficaces ne sont pas ceux mentionnés dans le brevet US précité, mais ceux choisis parmi les esters répondant à la formule générale:

R¹CO₂R² (I)

dans laquelle R¹ représente un radical hydrocarboné, linéaire ou ramifié, contenant de 1 à 4 atomes de carbone, éventuellement insaturé et R² représente un radical hydrocarboné contenant de 2 à 8 atomes de carbone, linéaire, ramifié ou cyclique, éventuellement insaturé.

L'invention a donc pour objet une composition à base de DMDS caractérisée en ce qu'elle contient, en poids, au moins 95 % de diméthyldisulfure, moins de 500 ppm de méthylmercaptan (MM), moins de 100 ppm de diméthylsulfure (DMS) et jusqu'à 1% d'au moins un agent masquant d' odeur choisi parmi la vanilline, l'éthylvanilline et, de préférence, les esters de formule générale (I).

Toute méthode connue de l'homme de l'art pour obtenir un DMDS aux teneurs réduites en impuretés volatiles telles que le MM et le DMS pourra être utilisée dans le cadre de la présente invention. Cependant, dans le cas d'un DMDS contenant des teneurs élevées en MM et DMS, une méthode particulièrement préférée consiste en un étêtage par distillation. Cette méthode présente l'avantage d'éliminer conjointement le MM et le DMS alors que les méthodes habituelles de réduction d'odeur, généralement basées sur l'élimination des mercaptans résiduels par réaction spécifique de la fonction mercaptan avec un agent d'élimination tel qu'une base ou un oxyde d'alcène en présence d'une base, sont sans effet sur le DMS présent dans le DMDS.

Le DMDS ainsi étêté, qui contient de préférence moins de 200 ppm de MM et moins de 50 ppm de DMS, est utilisé pour préparer une composition selon l'invention par simple addition d'au moins un agent masquant d'odeur.

L'un des avantages principaux du DMDS dans ses applications étant sa teneur en soufre élevée (68%), une teneur trop importante d'agent masquant d'odeur dans la composition conduirait à baisser ce titre en soufre et diminuerait l'intérêt de ce produit dans ses principales applications. La teneur maximale en agent(s) masquant d'odeur est donc fixée à 1 %, mais cette teneur est de préférence comprise entre 0,1 et 0,5 % et plus particulièrement égale à 0,2 %.

Comme exemples illustratifs mais non limitatifs d'esters de formule générale (I), on peut citer les acétates de butyle, d'isoamyle ou de benzyle et les butyrates d'éthyle, de propyle, de butyle, de 2-méthylbutyle ou d'isoamyle. Sont plus particulièrement préférés l'acétate d'isoamyle, lé butyrate de 2-méthylbutyle, le butyrate d'isoamyle, l'acétate de benzyle et les mélanges de ces composés. Les esters (I) peuvent être associés ou non à des orthophtalates répondant à la formule générale: 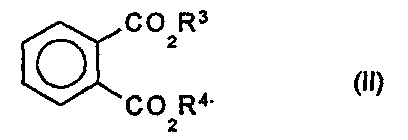 dans laquelle les symboles R³ et R⁴, identiques ou différents, représentent chacun un radical hydrocarboné contenant de 1 à 8 atomes de carbone, linéaire, ramifié ou cyclique, éventuellement insaturé. A titre d'exemple non limitatif de composé (ll), on peut mentionner plus particulièrement l'orthophtalate de diéthyle.

Une composition typique de la présente invention comprend en poids:

| | |
|---|---|
| acétate d'isoamyle | 0,1 % |
| orthophtalate de diéthyle | 0,1 % |
| DMDS étêté | 99,8 % |

Une autre composition typique de la présente invention comprend en poids:

| | |
|---|---|
| acétate d'isoamyle | 0,05 % |
| butyrate de 2-méthylbutyle | 0,03 % |
| acétate de benzyle | 0,02 % |
| orthophtalate de diéthyle | 0,1 % |
| DMDS étêté | 99,8 % |

Les exemples suivants illustrent l'invention sans la limiter.

### EXEMPLE 1 : Synthèse du diméthyldisulfure selon le procédé décrit dans le brevet EP 0 446 109

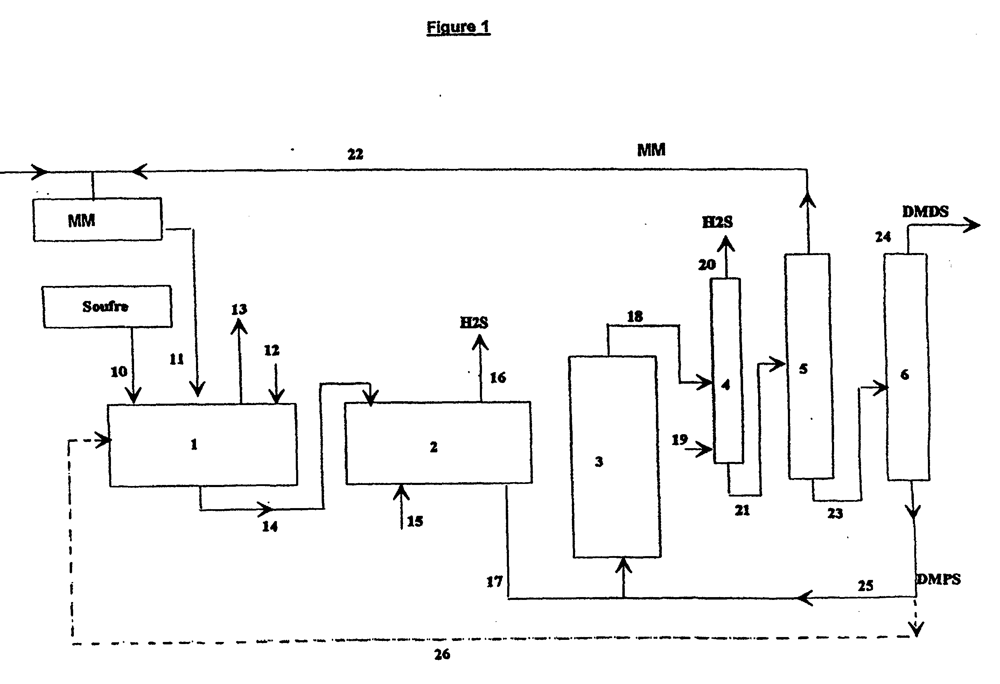

a) Appareillage La figure 1 annexée est un schéma de l'installation utilisée, associant deux réacteurs (réacteur primaire 1 et réacteur finisseur 3). Le réacteur primaire est un réacteur agité et le réacteur finisseur est un réacteur tubulaire à lit fixe. Entre ces deux réacteurs, est disposé un système de dégazage constitué d'un récipient à double enveloppe 2 muni d'un agitateur et surmonté d'une colonne refroidie qui permet de recondenser le méthylmercaptan qui peut être entraîné avec l'hydrogène sulfuré devant être éliminé. Cet équipement est complété par une pompe placée entre la sortie du dégazeur 2 et l'entrée du réacteur finisseur 3, qui permet d'alimenter ce réacteur en produit liquide traité dans le dégazeur. Une colonne de dégazage 4 sert à éliminer complètement l'H₂S dissous dans le liquide sortant du réacteur 3. Une colonne de distillation 5 permet de séparer la majeure partie du méthylmercaptan en excès en vue de son recyclage par la conduite 22 au réacteur 1. La colonne 6 permet de séparer les diméthylpolysulfures résiduels (DMPS) en vue de leur recyclage dans le réacteur 3 ou le réacteur 1.
b) Mode opératoire : Le méthylmercaptan (MM) est introduit liquide sous pression par la conduite 11 avec un débit de 960 g/h dans le réacteur 1. Le soufre liquide est introduit par la conduite 10 avec un débit de 160 g/h dans le réacteur 1 (MM/S = 4 molaire). Le réacteur 1 (volume réactionnel: 300 ml) contient 20 g de résine Ambertyst A21 sèche. La pression opératoire est maintenue à 5,5 bars relatifs et la température à 40°C. Le mélange réactionnel en sortie de réacteur 1 a la composition pondérale suivante hors méthylmercaptan en excès et hors H₂S : DMDS 85 %, DMPS 15 %. Ce mélange réactionnel est alors envoyé par la conduite 14 dans le dégazeur 2 pour être traité. Après traitement, le mélange débarrassé de l'H₂S est envoyé par la conduite 17 dans le réacteur finisseur 3 qui contient une charge de 94 g de résine A21 sèche. La pression dans le réacteur est de 5,5 bars relatifs et la température de 40°C. A la sortie du réacteur 3, le mélange a la composition pondérale suivante hors H₂S et hors méthylmercaptan en excès : DMDS 98,5 %, DMPS 1,5 %. Le mélange est alors introduit par le conduite 18 dans le dégazeur 4 pour l'élimination de l'H₂S qui s'est formé dans le réacteur 3 lors de la rétrogradation des diméthylpolysulfures par le méthylmercaptan pour donner du DMDS.

A la sortie de la colonne de dégazage 4, le mélange est introduit dans la première colonne de distillation 5 par la conduite 21 pour éliminer la quasi totalité du méthylmercaptan en excès. Ce méthylmercaptan peut être recyclé par la conduite 22 à l'introduction des réactifs dans le réacteur 1. A la sortie de la colonne 5, le mélange est amené par la conduite 23 dans la deuxième colonne de distillation 6 où les DMPS sont éliminés en fond de colonne par la conduite 25 pour être éventuellement recyclés dans le réacteur 3 ou par la conduite 26 pour être éventuellement recyclés dans le réacteur 1.

Le DMDS, finalement recueilli en tête de la colonne 6 par la conduite 24 et appelé **A**_{**o**} pour les tests olfactifs décrits dans les exemples suivants, a la composition pondérale suivante :
- DMDS: 99,3 %
- DMPS: 3000 ppm
- MM: 4000 ppm
- DMS : 300 ppm

### EXEMPLE 2 : Purification du diméthyldisulfure préparé selon le procédé décrit dans le brevet EP 0446 109

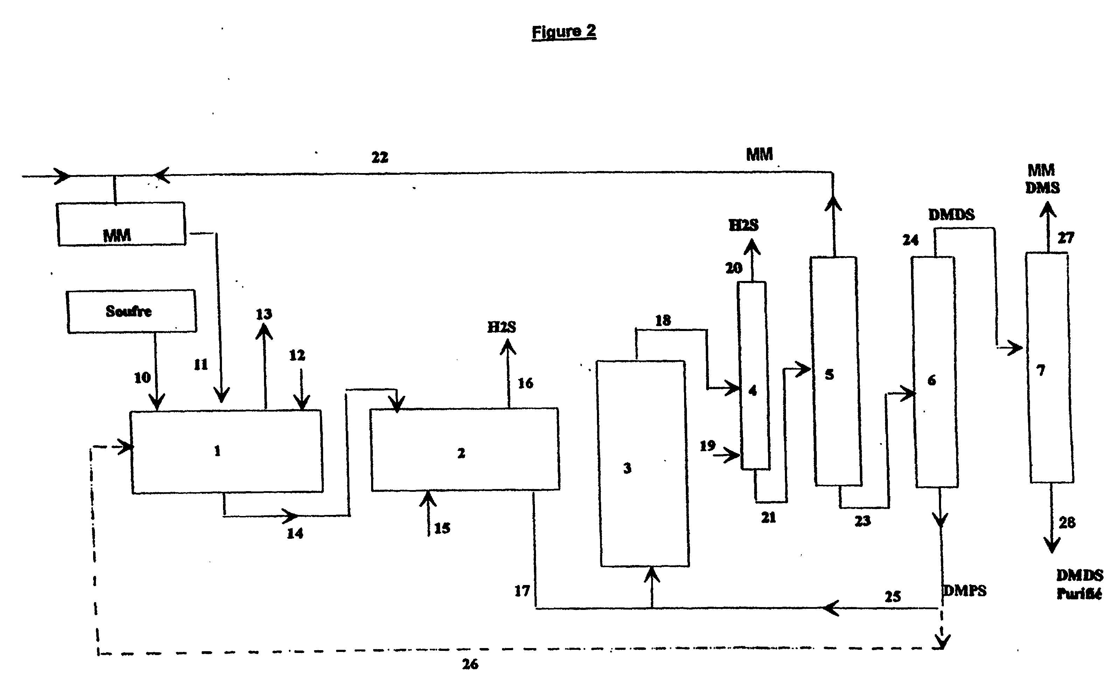

Le mode opératoire de synthèse est le même que celui décrit dans l'exemple 1 sauf que le DMDS sortant de la colonne 6 par la conduite 24 est introduit dans une troisième colonne de distillation 7 (voir schéma de la figure 2 annexée) où les impuretés volatiles telles que le méthylmercaptan et le diméthylsulfure sont éliminés en tête de colonne par la conduite 27. Le DMDS recueilli en fond de colonne par la conduite 28 a la composition pondérale suivante:
- DMDS: 99,7 %
- DMPS: 3000 ppm
- MM : < 100 ppm
- DMS : < 50 ppm

Ce DMDS purifié, ci-après appelé **B**_{**o**}, et un échantillon de DMDS **A**_{**o**} préparé à l'exemple 1, ont été soumis à un test olfactif. Les 8 personnes conviées à ce test ont unanimement reconnu une nette amélioration de l'odeur du DMDS **B**_{**o**} comparativement au DMDS **A**_{**o**}, mais toutes ont également signalé qu'il subsistait une odeur aillée et éthérée dans le DMDS **B**_{**o**}.

### EXEMPLE 3

A 100 g de DMDS **B**_{**o**} préparé dans l'exemple 2, on a ajouté 2000 ppm en poids de vanilline (4-hydroxy-3-méthoxybenzaldéhyde). La dissolution complète de la vanilline a été observée au bout d'une heure à 25°C. L'échantillon résultant a été appelé **B**_{**1**}.

### EXEMPLE 4

La vanilline utilisée dans l'exemple 3 a été remplacée par 2000 ppm d'éthyl-vanilline (3-éthoxy-4-hydroxybenzaldéhyde). Sa dissolution a été observée au bout d'une heure à 25°C. L'échantillon résultant a été appelé **B**_{**2**}.

Les exemples 5 et 6 illustrent la préparation de compositions à base de DMDS à l'odeur masquée par les produits préférés de la présente invention.

### EXEMPLE 5

A 100 g de DMDS **B**_{**o**} préparé dans l'exemple 2, on a ajouté 2000 ppm d'un mélange constitué en poids de 50 % d'acétate d'isoamyle et 50 % d'orthophtalate de diéthyle. Ce mélange étant liquide, la dissolution a été immédiate à 25°C. L'échantillon résultant a été appelé **B**_{**3**}.

### EXEMPLE 6

Le mélange utilisé dans l'exemple 5 a été remplacé par 2000 ppm d'un mélange ayant la composition pondérale suivante :

| | |
|---|---|
| acétate d'isoamyle | 25 % |
| orthophtalate de diéthyle | 50 % |
| butyrate de 2-méthylbutyle | 15 % |
| acétate de benzyle | 10 % |

La dissolution de ce mélange dans **Bo** a été immédiate à 25°C. L'échantillon résultant a été appelé **B**_{**4**}.

Les échantillons **B**_{**o**}, **B**_{**1**}**, B**_{**2**}**, B**_{**3**} et **B**_{**4**} ont été soumis à un test olfactif comparatif réalisé par le panel des 8 personnes mentionnées dans l'exemple 2. Il a été demandé à ces 8 personnes d'attribuer aux échantillons une note allant de 0 à 5 en fonction de leur préférence vis-à-vis de l'odeur, la note 0 étant attribuée à l'odeur la moins préférée, la note 5 à l'odeur la plus préférée et les notes 1, 2, 3, 4 leur permettant de classer les niveaux intermédiaires. Les résultats sont représentés dans le tableau suivant:

| **Echantillon Personne** | **B**_{**o**} | **B**_{**1**} | **B**_{**2**} | **B**_{**3**} | **B**_{**4**} |
|---|---|---|---|---|---|
| 1 | 0 | 2 | 3 | 4 | 5 |
| 2 | 1 | 2 | 4 | 3 | 5 |
| 3 | 0 | 3 | 2 | 4 | 5 |
| 4 | 0 | 2 | 3 | 4 | 5 |
| 5 | 0 | 3 | 2 | 4 | 5 |
| 6 | 0 | 2 | 3 | 4 | 5 |
| 7 | 1 | 2 | 4 | 3 | 5 |
| 8 | 1 | 2 | 3 | 5 | 4 |
| **Note sur 40** | **3/40** | **18/40** | **24/40** | **31/40** | **39/40** |

On remarquera que l'odeur des compositions **B**_{**1**}**, B**_{**2**}**, B**_{**3**} et **B**_{**4**} est toujours préférée à celle de **B**_{**o**} et que la note obtenue pour **B**_{**4**} est très proche du maximum possible (40). Les membres du panel ont en outre précisé qu'ils préféraient la note "fruitée" des compositions **B**_{**3**} et **B**_{**4**} à la note "vanillée-aillée" des compositions **B**_{**1**} et **B**_{**2**}**.**

Les exemples comparatifs 7 à 11 illustrent la nécessité d'éliminer la majeure partie des impuretés volatiles du DMDS pour observer un effet masquant d'odeur significatif.

### EXEMPLE 7

On a répété l'exemple 3 (masquant : vanilline) mais en remplaçant les 100 g de DMDS **B**_{**0**} par 100 g du DMDS non étêté **A**_{**0**} préparé dans l'exemple 1. L'échantillon résultant a été appelé **A**_{**1**}.

Les 8 membres du panel ont comparé l'odeur de **A**_{**1**} avec celle de **B**_{**o**} et tous ont préféré l'odeur de **B**_{**o**} (DMDS purifié de ses impuretés volatiles et sans agent masquant) à celle de la composition **A**_{**1**} à base de DMDS non purifié et de vanilline.

### EXEMPLE 8

On a suivi la procédure de l'exemple 7 en remplaçant la vanilline par l'éthyl-vanilline. Les 8 personnes du panel ont préféré l'odeur de l'échantillon **B**_{**o**} à celle de l'échantillon résultant (**A**_{**2**}).

### EXEMPLE 9

On a suivi la procédure de l'exemple 7 en remplaçant la vanilline par le menthol. L'odeur de l'échantillon **B**_{**o**} a encore été préférée à celle de l'échantillon résultant (**A**_{**3**}).

### EXEMPLE 10

On a suivi la procédure de l'exemple 7 en remplaçant la vanilline par le mélange de produits masquants mentionné dans l'exemple 5. L'échantillon résultant a été appelé **A**_{**4**}. Les 8 personnes du panel ont préféré l'odeur de **B**_{**o**} à celle de **A**_{**4**}.

### EXEMPLE 11

On a suivi la procédure de l'exemple 7 en remplaçant la vanilline par le mélange de produits masquants mentionné dans l'exemple 6. L'échantillon résultant a été appelé **A**_{**5**}. L'odeur de l'échantillon **B**_{**o**} a encore été préférée à celle de l'échantillon **A**_{**5**}.

## Revendications

1. Composition à base de diméthyldisulfure (DMDS) **caractérisée en ce qu'**elle contient, en poids, au moins 95 % de DMDS, moins de 500 ppm de méthylmercaptan, moins de 100 ppm de diméthylsulfure et jusqu'à 1 % d'au moins un agent masquant d'odeur choisi parmi la vanilline, l'éthylvalline et les esters répondant à la formule générale:
R¹CO₂R² (I)
dans laquelle R¹ représente un radical hydrocarboné, linéaire ou ramifié, contenant de 1 à 4 atomes de carbone, éventuellement insaturé et R² représente un radical hydrocarboné contenant de 2 à 8 atomes de carbone, linéaire, ramifié ou cyclique, éventuellement insaturé.

2. Composition selon la revendication 1 contenant moins de 200 ppm de méthylmercaptan et moins de 50 ppm de diméthylsulfure.

3. Composition selon la revendication 1 ou 2 contenant de 0,1 à 0,5 % d'agent(s) masquant d'odeur, de préférence 0,2 %.

4. Composition selon l'une des revendications 1 à 3 dans laquelle l'agent masquant d'odeur est choisi parmi les asters de formule (I),

5. Composition selon la revendication 4 dans laquelle l'agent masquant d'odeur est choisi dans le groupe constitué par l'acétate d'isoamyle, le butyrate de 2-méthylbutyle, le butyrate d'isoamyle, l'acétate de benzyle et les mélanges de ces composés.

6. Composition selon la revendication 4 ou 5 dans laquelle l'ester de formule (I) est associé à un orthophtalate répondant à la formule générale: 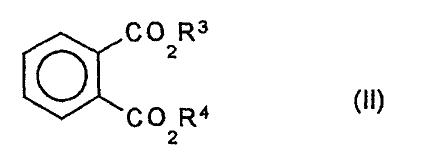 dans laquelle les symboles R³ et R⁴, identiques ou différents, représentent chacun un radical hydrocarboné contenant de 1 à 8 atomes de carbone, linéaire, ramifié ou cyclique, éventuellement insaturé.

7. Composition selon la revendication 6 dans laquelle l'orthophtalate est l'orthophtalate de diéthyle.

8. Composition selon la revendication 7 contenant 0,1 % d'acétate d'isoamyle et 0,1 % d'orthophtalate de diéthyle.

9. Composition selon la revendication 7 contenant 0,05 % d'acétate d'isoamyle, 0,03 % de butyrate de 2-méthylbutyle, 0,02 % d'acétate de benzyle et 0,1 % d'orthophtalate de diéthyle.

## Claims

1. Composition based on dimethyl disulphide (DMDS), **characterized in that** it comprises, by weight, at least 95% of DMDS, less than 500 ppm of methyl mercaptan, less than 100 ppm of dimethyl sulphide and up to 1% of at least one odour-masking agent chosen from vanillin, ethylvanillin and the esters corresponding to the general formula:
R¹CO₂R² (I)
in which R¹ represents an optionally unsaturated, linear or branched hydrocarbon-comprising radical comprising from 1 to 4 carbon atoms and R² represents an optionally unsaturated, linear, branched or cyclic hydrocarbon-comprising radical comprising from 2 to 8 carbon atoms.

2. Composition according to Claim 1, comprising less than 200 ppm of methyl mercaptan and less than 50 ppm of dimethyl sulphide.

3. Composition according to Claim 1 or 2, comprising from 0.1 to 0.5% of odour-masking agent (s), preferably 0.2%.

4. Composition according to one of Claims 1 to 3, in which the odour-masking agent is chosen from the esters of formula (I).

5. Composition according to Claim 4, in which the odour-masking agent is chosen from the group consisting of isoamyl acetate, 2-methylbutyl butyrate, isoamyl butyrate, benzyl acetate and the mixtures of these compounds.

6. Composition according to Claim 4 or 5, in which the ester of formula (I) is used in combination with an orthophthalate corresponding to the general formula: 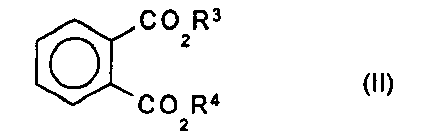 in which the symbols R³ and R⁴, which are identical or different, each represent an optionally unsaturated, linear, branched or cyclic hydrocarbon-comprising radical comprising from 1 to 8 carbon atoms.

7. Composition according to Claim 6, in which the orthophthalate is diethyl orthophthalate.

8. Composition according to Claim 7, comprising 0.1% of isoamyl acetate and 0.1% of diethyl orthophthalate.

9. Composition according to Claim 7, comprising 0.05% of isoamyl acetate, 0.03% of 2-methylbutyl butyrate, 0.02% of benzyl acetate and 0.1% of diethyl orthophthalate.

## Patentansprüche

1. Zusammensetzung auf Basis von Dimethyldisulfid (DMDS), **dadurch gekennzeichnet, daß** sie gewichtsbezogen mindestens 95 % DMDS, weniger als 500 ppm Methylmercaptan, weniger als 100 ppm Dimethylsulfid und bis zu 1 % mindestens eines geruchsmarkierenden Mittels enthält, das ausgewählt ist aus Vanillin, Ethylvanillin und Estern der allgemeinen Formel
R¹CO₂R² (I),
in der R¹ einen gegebenenfalls ungesättigten, linearen oder verzweigten Kohlenwasserstoffrest mit 1 bis 4 Kohlenstoffatomen darstellt und R² einen gegebenenfalls ungesättigten, linearen, verzweigten oder ringförmigen Kohlenwasserstoffrest mit 2 bis 8 Kohlenstoffatomen darstellt.

2. Zusammensetzung nach Anspruch 1, enthaltend weniger als 200 ppm Methylmercaptan und weniger als 50 ppm Dimethylsulfid.

3. Zusammensetzung nach Anspruch 1 oder 2, enthaltend 0,1 bis 0,5 %, vorzugsweise 0,2 %, geruchsmarkierende(s) Mittel.

4. Zusammensetzung nach einem der Ansprüche 1 bis 3, wobei das geruchsmarkierende Mittel ausgewählt ist aus Estern der Formel (I).

5. Zusammensetzung nach Anspruch 4, wobei das geruchsmarkierende Mittel ausgewählt ist aus der Gruppe von Isoamylacetat, 2-Methylbutylbutyrat, Isoamylbutyrat, Benzylacetat und Mischungen dieser Verbindungen.

6. Zusammensetzung nach Anspruch 4 oder 5, wobei der Ester der Formel (I) gemeinsam mit einem Orthophthalat der allgemeinen Formel (II) vorliegt 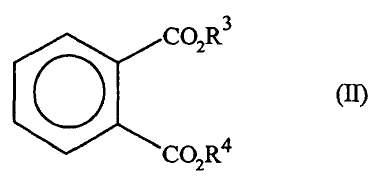 wobei die Symbole R³ und R⁴, identisch oder verschieden, jeweils einen gegebenenfalls ungesättigten, linearen, verzweigten oder ringförmigen Kohlenwasserstoffrest mit 1 bis 8 Kohlenstoffatomen darstellen.

7. Zusammensetzung nach Anspruch 6, wobei das Orthophthalat ein Diethylorthophthalat ist.

8. Zusammensetzung nach Anspruch 7, enthaltend 0,1 % Isoamylacetat und 0,1 % Diethylorthophthalat.

9. Zusammensetzung nach Anspruch 7, enthaltend 0,05 % Isoamylacetat, 0,03 % 2-Methylbutylbutyrat, 0,02 % Benzylacetat und 0,1 % Diethylorthophthalat.
